# EUROPEAN PATENT APPLICATION

(11) **EP 4 729 530 A1**
(43) Date of publication of application: **22.04.2026**
(21) Application number: 24823733.1
(22) Date of filing: 13.06.2024
(51) Int. Cl.: C07K 7/08, A61P 25/28, A23L 33/18, A61K 38/00, A61P 25/00

(54) **NOVEL PEPTIDE AND USE THEREOF**

(30) Priority: 15.06.2023 KR 20230076674; 12.06.2024 KR 20240076255
(71) Applicant: Gemvax & Kael Co., Ltd., Daejeon 34036 (KR)
(72) Inventor: KIM, Sang Jae, Seoul 06360 (KR)
(74) Representative: Stolmár & Partner Patentanwälte PartG mbB
(86) International application number: PCT/KR2024/008142
(87) International publication number: WO 2024/258216

(57) **Abstract**

The present invention relates to: a peptide which has the effects of preventing, alleviating, and/or treating 4R tauopathy, and which is safe with respect to the human body, and thus has reduced side effects including adverse reactions; and a pharmaceutical composition and a health functional food, which include same.

## Description

### [Technical Field]

The present invention relates to a novel peptide and the use thereof.

### [Background Art]

Tau is abundantly expressed in neurons of the central nervous system (CNS) and primarily functions to maintain the stability of axonal microtubules.

When tau undergoes hyperphosphorylation, in which excessive phosphate groups are attached to the tau protein, it detaches from microtubules and forms insoluble aggregates known as neurofibrillary tangles (NFTs). Neurodegenerative diseases associated with such abnormal tau aggregation are collectively referred to as tauopathies.

Tauopathies are classified into 3R and 4R tauopathies depending on the tau protein isoform involved.

4R tauopathies include progressive supranuclear palsy (PSP), corticobasal degeneration (CBD), argyrophilic grain disease (AGD), globular glial tauopathy (GGT), and aging-related tau astrogliopathy (ARTAG).

The number of patients with 4R tauopathies is increasing with aging, but currently, no medications are available to effectively treat or prevent 4R tauopathies.

Although phase 3 clinical trials are ongoing for LMTM, which is known as a tau aggregation inhibitor, its specific efficacy for 4R tauopathies has not been reported. In addition, LMTM exhibits cytotoxicity (GI₅₀ = 6.4±0.3 µM) within the tau aggregation inhibition range (EC₅₀ = 2.2±0.2 µM).

Therefore, as a result of extensive studies to treat 4R tauopathy by inhibiting tau hyperphosphorylation and aggregation, the inventors of the present invention have completed the present invention by developing a novel peptide.

### [Summary of Invention]

### [Problems to be Solved by Invention]

An object of the present invention is to provide a novel peptide that exhibits efficacy for preventing or treating 4R tauopathy.

Another object of the present invention is to provide a pharmaceutical composition for preventing or treating 4R tauopathy.

Yet another object of the present invention is to provide a health functional food for preventing or alleviating 4R tauopathy.

### [Means for Solving Problems]

1. A peptide of Formula 1 below or a pharmaceutically acceptable salt thereof: 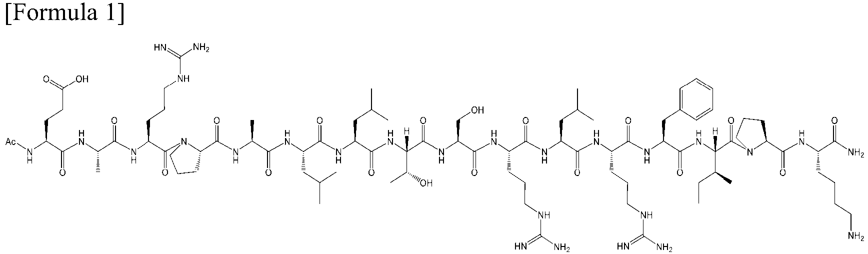
2. A pharmaceutical composition for preventing or treating 4R tauopathy, including the peptide of the above 1 or a pharmaceutically acceptable salt thereof.
3. The pharmaceutical composition according to the above 2, wherein the 4R tauopathy is any one selected from the group consisting of progressive supranuclear palsy (PSP), corticobasal degeneration (CBD), argyrophilic grain disease (AGD), globular glial tauopathy (GGT), and aging-related tau astrogliopathy (ARTAG).
4. The pharmaceutical composition according to the above 3, wherein the prevention or treatment of 4R tauopathy is achieved by inhibiting hyperphosphorylation and aggregation of tau protein.
5. A health functional food for preventing or alleviating 4R tauopathy, including the peptide of Formula 1 below or a food-acceptable salt thereof: 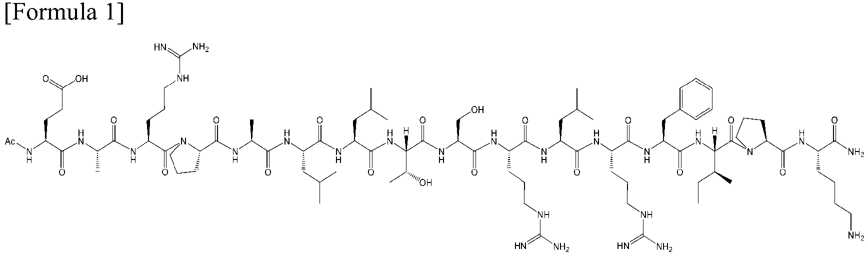
6. The health functional food according to the above 5, wherein the 4R tauopathy is any one selected from the group consisting of progressive supranuclear palsy, corticobasal degeneration, argyrophilic grain disease, globular glial tauopathy, and aging-related tau astrogliopathy.
7. The health functional food according to the above 6, wherein the prevention or alleviation of 4R tauopathy is achieved by inhibiting hyperphosphorylation and aggregation of tau protein.

### [Advantageous effects]

The peptide of Formula 1 of the present invention and the pharmaceutically acceptable salt thereof exhibit excellent tau protein hyperphosphorylation inhibitory effects.

The peptide of Formula 1 of the present invention and the pharmaceutically acceptable salt thereof exhibit excellent tau protein aggregation inhibitory effects.

The pharmaceutical composition and the health functional food including the peptide of Formula 1 of the present invention or the salt thereof may exhibit preventive, alleviating, and/or therapeutic effects for 4R tauopathy by inhibiting tau protein hyperphosphorylation and aggregation.

The peptide, the pharmaceutical composition, and the health functional food of the present invention may exhibit stable, long-lasting effects in vivo.

### [Brief Description of Drawings]

FIG. 1 illustrates the results of an experiment confirming the stability of GV2002 in human plasma.
FIG. 2 illustrates the results of the pharmacokinetic (PK) profile of GV2002 administered intravenously to rats.
FIG. 3 illustrates the results of the pharmacokinetic profile of GV2002 administered subcutaneously to rats.
FIG. 4 illustrates the mouse groups used in the in vivo experiment to verify the efficacy of GV2002.
FIG. 5 illustrates the schedule of the efficacy evaluation experiment of GV2002 conducted in a 4R tauopathy mouse model.
FIGS. 6 and 7 illustrate the results of an experiment evaluating motor ability improvement in a 4R tauopathy mouse model.
FIG. 8 illustrates the method and results of an experiment evaluating cognitive ability improvement in a 4R tauopathy mouse model.
FIG. 9 illustrates the experimental method and results of an experiment evaluating improvement in spatial memory ability in a 4R tauopathy mouse model.
FIG. 10 illustrates the results of an experiment evaluating the tau hyperphosphorylation inhibitory effect of GV2002 using brain tissue immunofluorescence staining.
FIG. 11 illustrates the results of an experiment evaluating the tau oligomer formation inhibitory effect of GV2002 using brain tissue Tau-BiFC imaging.
FIG. 12 illustrates the results of an experiment evaluating the tau hyperphosphorylation and aggregation inhibitory effect of GV2002 using brain lysate analysis.

### [Mode for Carrying out Invention]

The present invention provides a peptide of Formula 1 below or a pharmaceutically acceptable salt thereof:

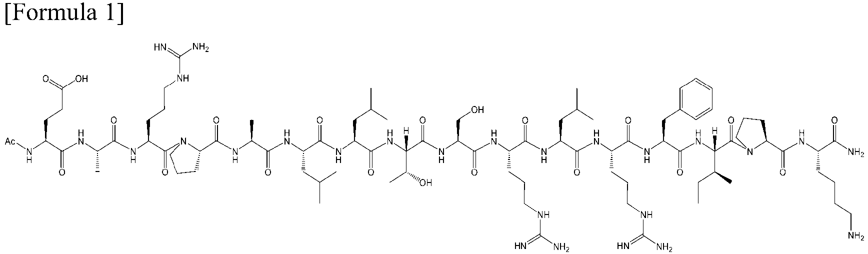

In the present invention, the peptide of Formula 1 includes functional equivalents thereof. The term "functional equivalent" refers to a peptide exhibiting substantially the same physiological activity as the peptide of Formula 1.

The present invention provides a pharmaceutical composition for preventing or treating 4R tauopathy, including a peptide of Formula 1 or a pharmaceutically acceptable salt thereof as an active ingredient.

As used herein, the term "pharmaceutically acceptable" refers to a property that is non-toxic to cells or organisms exposed to the composition.

As used herein, the term "4R tauopathy" collectively refers to neurodegenerative diseases caused by the aggregation of 4R tau protein, including progressive supranuclear palsy, corticobasal degeneration, argyrophilic grain disease, globular glial tauopathy, and aging-related tau astrogliopathy.

As used herein, the term "prevention" refers to any action that inhibits or delays the onset or progression of 4R tauopathy.

As used herein, the term "alleviation" and "treatment" refer to any action that alleviates or beneficially modifies the symptoms of a subject who has developed, or is at risk of developing, 4R tauopathy.

In one embodiment, the prevention, alleviation, and/or treatment may be achieved by inhibiting tau protein hyperphosphorylation.

In one embodiment, the prevention, alleviation, and/or treatment may be achieved by inhibiting tau protein aggregation.

In one embodiment, the prevention, alleviation, and/or treatment may be achieved by alleviating impairment in motor ability of a subject who has developed, or is at risk of developing, 4R tauopathy.

In one embodiment, the prevention, alleviation, and/or treatment may be achieved by alleviating impairment in cognitive ability of a subject who has developed, or is at risk of developing, 4R tauopathy.

In one embodiment, the prevention, alleviation, and/or treatment may be achieved by alleviating impairment in spatial memory ability of a subject who has developed, or is at risk of developing, 4R tauopathy.

The pharmaceutical composition of the present invention may include the active ingredient alone or may further include one or more pharmaceutically acceptable carriers, excipients, or diluents.

The carrier, excipient, or diluent that may be included in the pharmaceutical composition of the present invention may include lactose, dextrose, sucrose, dextrin, maltodextrin, sorbitol, mannitol, xylitol, erythritol, maltitol, starch, gum acacia, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, or mineral oil, but is not limited thereto.

The pharmaceutical composition of the present invention may be administered to a subject.

As used herein, the term "administration" refers to introducing a predetermined substance into a subject by an appropriate method, and the term "subject" refers to all animals that have developed or may develop 4R tauopathy, such as livestock and mice, and may be mammals, including humans, for example.

The administration route of the pharmaceutical composition of the present invention may be oral, intravenous, intramuscular, intra-arterial, intramedullary, intrathecal, intracardiac, transdermal, subcutaneous, intraperitoneal, intranasal, intestinal, topical, sublingual, or rectal, but is not limited thereto.

In one embodiment, the composition of the present invention may be administered orally or parenterally
When the composition of the present invention is administered parenterally, it is preferable to select an administration method such as topical application, intraperitoneal injection, intrarectal injection, subcutaneous injection, intravenous injection, intramuscular injection, or intrathoracic injection, but is not limited thereto.

The pharmaceutical composition of the present invention may be a solid preparation for oral administration, such as a tablet, pill, powder, granule or capsule.

The pharmaceutical composition of the present invention may be a liquid preparation for oral administration, such as a suspension, a solution, an emulsion or a syrup.

The pharmaceutical composition of the present invention may be a preparation for parenteral administration, such as a sterile aqueous solution, a non-aqueous solution, a suspension, an emulsion, a lyophilized preparation or a suppository.

The pharmaceutical composition of the present invention may be administered to a subject in a pharmaceutically effective amount, and the term "pharmaceutically effective amount" refers to an amount which is sufficient to provide a desired pharmacological effect at a reasonable benefit/risk ratio applicable to 4R tauopathy treatment.

The pharmaceutically effective amount of the pharmaceutical composition of the present invention may be determined based on factors including the severity of 4R tauopathy, the activity of the pharmaceutical composition, the sensitivity of the subject or patient to the pharmaceutical composition, the time of administration, the route of administration, the excretion rate, the duration of treatment, and concomitant medications, as well as other factors well known in the medical field, and may be appropriately selected by those skilled in the art.

The pharmaceutical composition of the present invention may be administered as a standalone therapeutic agent or in combination with other conventional therapeutic agents. When administered in combination, it may be administered sequentially or concurrently, and may be administered as a single dose or in multiple doses, and this may be easily determined by those skilled in the art.

The present invention provides a health functional food for preventing or alleviating 4R tauopathy, including a peptide of Formula 1 or a food-acceptable salt thereof.

The health functional food of the present invention refers to a food manufactured and processed using raw materials or ingredients having beneficial functionality for the human body, as defined by the Health Functional Food Act of Korea. The term "functionality" refers to ingestion for the purpose of obtaining beneficial effects for health use such as controlling nutrients related to the structure or function of the human body or physiological actions.

The health functional food of the present invention may include conventional food additives, and the suitability of such food additives shall be determined on the basis of the standards and specifications of the corresponding items according to the General Regulations and General Test Methods of the Food Additives Code approved by the Korean Ministry of Food and Drug Safety, unless otherwise specified.

The items listed in the Food Additives Code may include, for example: chemical synthetic substances such as ketones, glycine, potassium citrate, nicotinic acid, and cinnamic acid; natural additives such as gardenia blue pigment, licorice extract, crystalline cellulose, red yeast rice pigment, and guar gum; and mixed preparations such as L-glutamate sodium preparations, alkaline agents for noodles, preservative preparations, and tar color preparations, and the like.

The health functional food of the present invention may include a peptide of Formula 1 in an amount of 0.01 to 95% by weight, preferably 1 to 80% by weight, based on the total weight of the health functional food, for the purpose of preventing and/or alleviating 4R tauopathy. In addition, the health functional food may be manufactured and processed into tablets, capsules, powders, granules, liquids, pills, or the like, for the purpose of preventing and/or alleviating 4R tauopathy.

Hereinafter, the present invention will be described in detail by way of examples to further illustrate the present invention. However, the following examples are provided only to facilitate understanding of the present invention, and the scope of the present invention is not limited thereto.

### Examples

### [Example 1] Preparation of GV2002

A peptide (hereinafter referred to as GV2002), represented by the structural formula of Formula 1 below, was prepared.

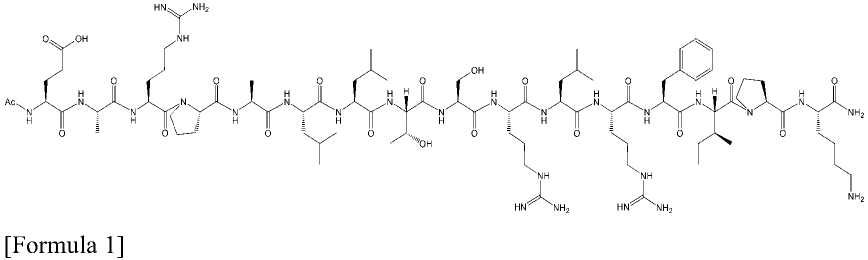

GV2002 was synthesized by sequentially coupling amino acids from the C-terminus using the Fmoc solid-phase peptide synthesis (SPPS) method known in the art.

All amino acid starting materials used for peptide synthesis were protected at the N-terminus with Fmoc, and all residues were protected with Boc, t-Bu (t-butylester), or Pbf (2,2,4,6,7-pentamethyl dihydro-benzofuran-5-sulfonyl), etc., which are removable under acidic conditions. For example, the amino acid starting materials used for peptide synthesis were as follows:

Fmoc-Ala-OH, Fmoc-Arg(Pbf)-OH, Fmoc-Glu(OtBu)-OH, Fmoc-Lys(Boc)-OH, Fmoc-Pro-OH, Fmoc-Leu-OH, Fmoc-Ile-OH, Fmoc-Phe-OH, Fmoc-Ser-OH, Fmoc-Thr-OH, Fmoc-Lys(Boc)-OH, Fmoc-Arg(Pbf)-OH.

As coupling reagents, diisopropylcarbodiimide (DIC), dimethylformamide (DMF), ethyl(hydroxyamino)cyanoacetate (Oxyma), and isopropyl alcohol (IPA) were used. Piperidine in DMF (20%) was used for Fmoc removal. A cleavage cocktail [95% trifluoroacetic acid (TFA)/5% water (H₂O)/50 mg/mL dithiothreitol (DTT)] was used to cleave the synthesized peptide from the resin and remove the protecting groups.

The N-terminus of the peptide was acetylated using acetic anhydride (Ac₂O), diisopropylethylamine (DIEA), and dimethylformamide (DMF) for N-terminal capping. The C-terminus of the peptide was converted into an amide.

The peptide was synthesized in a state in which the starting amino acid, bearing a protecting group, was first bound to a solid support, by sequentially coupling each subsequent amino acid, washing with a solvent, and removing the protecting group in a repeated manner. The synthesized peptide was cleaved from the resin, purified by HPLC, confirmed by MS, and lyophilized.

For the counter ion exchange process, GV2002, dissolved in water in the form of an HOAc salt, was converted into a chloride form using AmberChrom ion-exchange resin. The chloride-exchanged GV2002 was filtered through a PVDF filter and lyophilized.

The specific synthesis process for GV2002 is as follows:

### (1) Coupling

Amino acids were coupled sequentially from the C-terminus to the N-terminus to the Fmoc-Rink methylbenzhydrylamine (MBHA) resin by adding each amino acid together with the coupling reagents DIC/Oxyma/IPA dissolved in DMF. The resin was then washed with DMF.

### (2) Fmoc Deprotection and N-terminal Capping

Deprotection was performed by adding piperidine in DMF (20%) to remove the Fmoc protecting group, followed by acetylation of the N-terminus.

### (3) Cleavage

After completion of the peptide synthesis, the peptide was cleaved from the resin by treating the synthesized peptide-bound resin with a cleavage cocktail.

### (4) Filtration

The resulting mixture was concentrated and precipitated with MTBE/hexane. The precipitate was then filtered and dried.

### (5) Purification

The resulting dried filtrate was purified by preparative HPLC, the molecular weight was confirmed by LC/MS, and lyophilized.

### (6) Preparation of Powder

The lyophilized peptide was converted into a chloride form using AmberChrom ion-exchange resin, filtered through a PVDF filter, and lyophilized again to obtain the peptide in powder form.

### [Example 2] Confirmation of GV2002 Stability in Human Plasma

The peptide was added to human plasma and diluted to a concentration of 5 µM, followed by incubation at 37 °C for 10, 60, and 240 minutes with stirring. The reaction mixture was collected and pretreated by plasma protein precipitation using methanol containing 0.1% formic acid. The resulting supernatants were collected and analyzed by LC-MS under the conditions shown in Table 1 below to determine the percentage of residual peptide relative to the initial peptide amount.

**[TABLE 1]**

| | | | | |
|---|---|---|---|---|
| HPLC conditions | Column | ACQUITY UPLC^{®} BEH C₁₈ Column (2.10 mm x 50 mm, 1.7 µm) | | |
| | Column temperature | 35°C | | |
| | Mobile phase | Time (min) | A: 0.1% Formic acid in Water | B: 0.1% Formic acid in Acetonitrile |
| | | 0.00 | 95% | 5% |
| | | 0.02 | 95% | 5% |
| | | 2.80 | 5% | 95% |
| | | 3.20 | 5% | 95% |
| | | 3.50 | 95% | 5% |
| | | 5.00 | 95% | 5% |
| | Flow rate | 0.4 mL/min | | |
| | Dose volume | 2 µL | | |
| MS conditions | Ion source | ES + Source | | |
| | Capillary (kV) | 1.00 | | |
| | Desolvation Temperature | 500°C | | |
| | Gas flow | - Desolvation (L/hr): 800 - Con (L/hr): 150 | | |
| | Nebulizer | 7.0 bar | | |

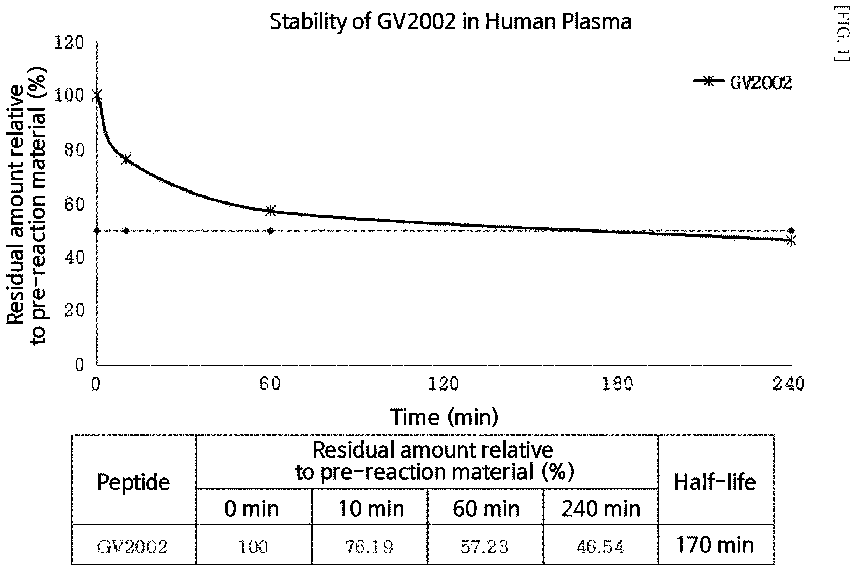

As shown in FIG. 1, the percentage of residual peptide relative to the peptide amount before reaction of GV2002 was 57.23% at 60 minutes and 46.54% at 240 minutes. This confirms that GV2002 exhibits stability in plasma.

### [Example 3] Pharmacokinetic Profile of GV2002

To determine the pharmacokinetic profile of GV2002 prepared in Example 1, plasma was collected from rats administered with GV2002. The collected plasma was pretreated by protein precipitation using nine times the volume of methanol containing 0.1% formic acid, followed by centrifugation at 16,100 g for 10 minutes. The resulting supernatant was collected and subjected to LC-MS analysis under the conditions shown in Table 2.

**[TABLE 2]**

| | | | | |
|---|---|---|---|---|
| HPLC conditions | Column | ACQUITY UPLC^{®} BEH C₁₈ Column (2.10 mm x 50 mm, 1.7 µm) | | |
| | Column temperature | 35°C | | |
| | Mobile phase | Time (min) | A: 0.1% Formic acid in Water | B: 0.1% Formic acid in Acetonitrile |
| | | 0.00 | 95% | 5% |
| | | 0.02 | 95% | 5% |
| | | 0.07 | 5% | 95% |
| | | 1.70 | 5% | 95% |
| | | 1.75 | 95% | 5% |
| | | 3.00 | 95% | 5% |
| | Flow rate | 0.5 mL/min | | |
| | Dose volume | 2 µL | | |
| MS conditions | Ion source ES + Source | | | |
| | Capillary (kV) | 1.00 | | |
| | Desolvation Temperature | 500°C | | |
| | Gas flow | - Desolvation (L/hr): 800 - Con (L/hr): 150 | | |
| | Nebulizer | 7.0 bar | | |

### 3-1. Pharmacokinetic Profile of GV2002 Through Intravenous Administration

GV2002 was administered intravenously (IV) to three Sprague Dawley rats per group. Blood samples were collected at 0.033, 0.083, 0.17, 0.25, 0.5, 1, and 2 hours after administration. The collected blood samples were centrifuged to separate plasma, pretreated, and analyzed using LC-MS. Pharmacokinetic parameters were calculated using the non-compartmental analysis model of the Phoenix WinNonlin (Pharsight, ver. 6.4, USA) program.

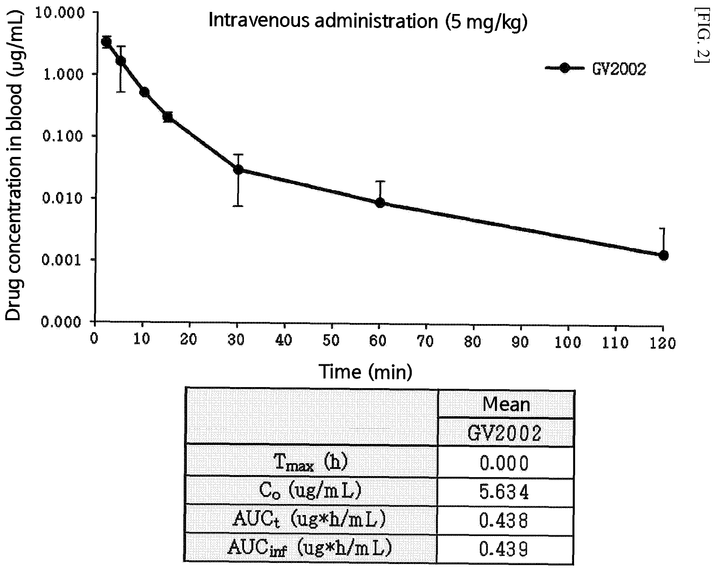

Consequently, as shown in FIG. 2, the AUCt value of GV2002 was 0.438 µg·h/mL. This confirms that GV2002 remains in a stable state in vivo without degradation and persists for a long period of time.

### 3-2. Pharmacokinetic Profile of GV2002 Through Subcutaneous Administration

GV2002 was administered subcutaneously (SC) to three Sprague Dawley rats per group. Blood samples were collected at 0.033, 0.083, 0.17, 0.25, 0.5, 1, and 2 hours after administration. The collected blood samples were centrifuged to separate plasma, pretreated, and analyzed using LC-MS. Pharmacokinetic parameters were calculated using the non-compartmental analysis model of Phoenix WinNonlin (Pharsight ver. 6.4, USA).

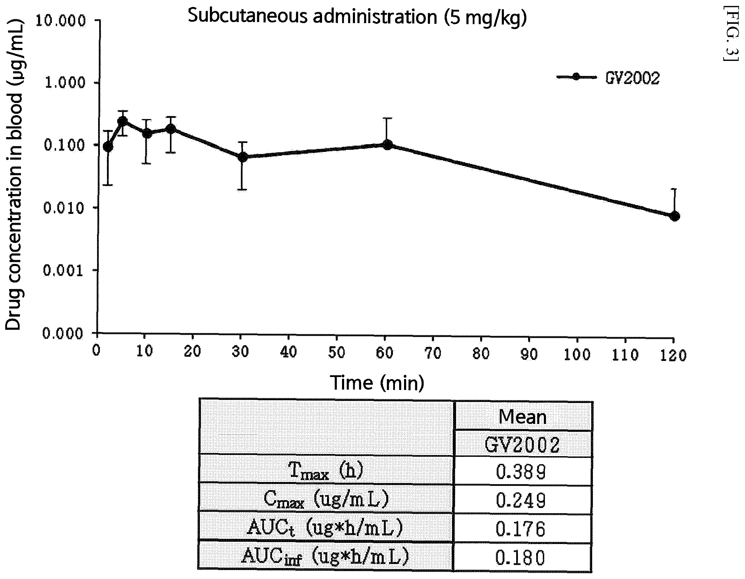

Consequently, as shown in FIG. 3, this confirms that GV2002 remains in a stable state in vivo without degradation and persists for a long period of time even after subcutaneous administration.

### [Example 4] GV2002 Efficacy Evaluation Based on a 4R Tauopathy Animal Model

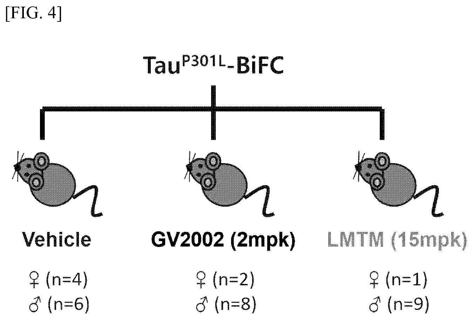

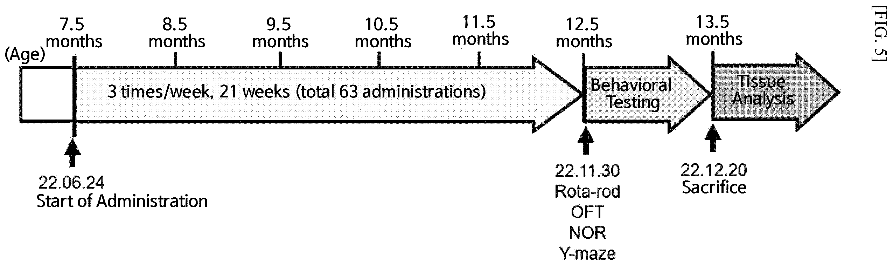

In vivo experiments were performed on the 4R tauopathy mouse groups shown in FIG. 4 according to the schedule shown in FIG. 5 and the outline in Table 3 below.

**[TABLE 3]**

| | |
|---|---|
| Animal | 4R Tau^{P301L}-BiFC mouse |
| Age | 7.5 months (at the time of first administration) |
| Test Compound | GV2002 (2 mg/kg, dissolved in saline) - subcutaneous administration |
| Reference Compound | LMTM (15 mg/kg, dissolved in saline) - oral administration |
| Vehicle | Saline-subcutaneous injection |
| Subcutaneous Injection Volume | < 5 mL/kg |
| | Male / 130 µL (average body weight: 33.2 g) |
| | Female / 110 µL (average body weight: 27.0 g) |
| Dosing Period | 3 times/week, 21 weeks (total 63 administrations) |
| Behavioral Testing | ▪ Rota-rod test |
| | ▪ Open Field Test (OFT) |
| | ▪ Novel Object Recognition Test (NOR test) |
| | ▪ Y-maze test |
| Brain Tissue Analysis | ▪ Immunofluorescence staining of brain tissue |
| | ▪ Brain lipid staining (Sudan Black B stain) |
| Brain Lysate Analysis | Tau immunoblot |

### 4-1. Evaluation of the Alleviation of Impairment in Motor Ability through the Rota-rod Experiment

Rota-rod experiments were conducted on mice with 4R tauopathy treated with GV2002 for five months, from 7.5 to 12.5 months of age, to evaluate the efficacy of GV2002 in alleviating impairment in motor ability.

### (1) Experimental Method

On day 1, acclimation training was conducted, and on day 2, behavioral testing was conducted. Prior to acclimation training and behavioral testing, mice were allowed to acclimate to the testing environment for one hour. During acclimation training, mice walked on a treadmill at a speed ranging from 5 to 25 rpm for 300 seconds, and any mouse that lost its balance and fell to the floor was allowed to walk again. In the behavioral testing, mice walked on a treadmill at a speed that gradually increased from 5 rpm to 40 rpm for 300 seconds, and the time (in seconds) until each mouse lost its balance and fell to the floor was measured. The measured values were analyzed using a two-way ANOVA with Dunnett's multiple comparisons test.

### (2) Experimental Results

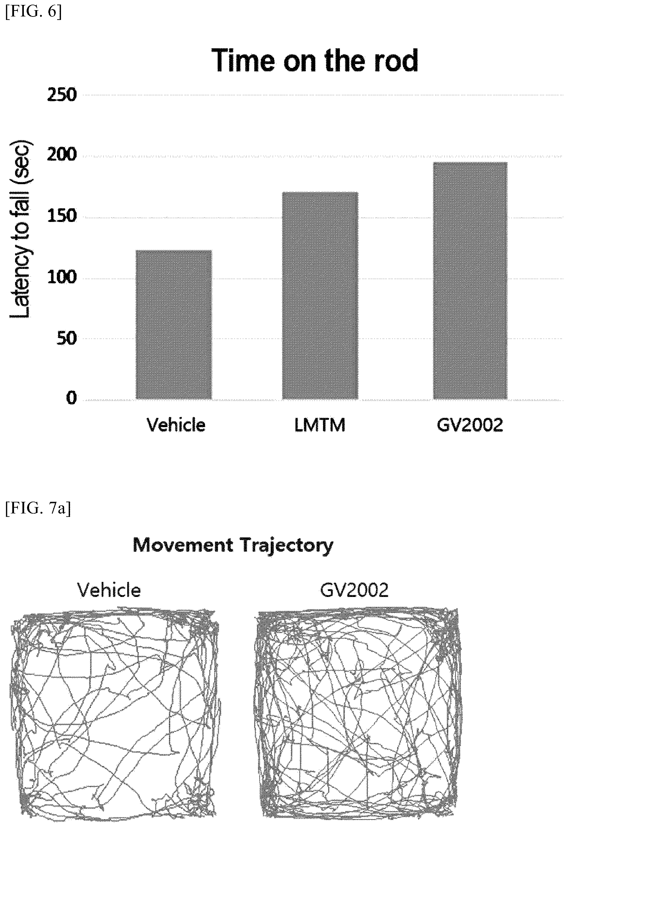

As shown in FIG. 6, the GV2002-administered group exhibited significantly improved motor ability compared to the vehicle-administered group and showed relatively enhanced motor ability compared to the reference compound LMTM-administered group.

### 4-2. Evaluation of the Alleviation of Impairment in Motor Ability through the Open Field Experiment

Open field experiments were conducted on mice with 4R tauopathy administered GV2002 for five months, from 7.5 to 12.5 months of age, to evaluate the efficacy of GV2002 in alleviating impairment in motor ability.

### (1) Experimental Method

To prevent changes in mouse behavioral responses, handling was performed for five days (days 1 to 5) prior to the experiment. Each handling session lasted two minutes. On day 6, mice were placed in a 40 cm × 40 cm enclosure and subjected to an open field experiment for 10 minutes. Prior to the experiment, mice were allowed one hour to acclimate to the testing environment. During the open field experiment, movement trajectories, distance traveled, and movement speed of the mice were recorded. The illumination level was maintained at a constant level throughout the experiment. The recorded values were analyzed using one-way ANOVA with Dunnett's multiple comparisons test.

### (2) Experimental Results

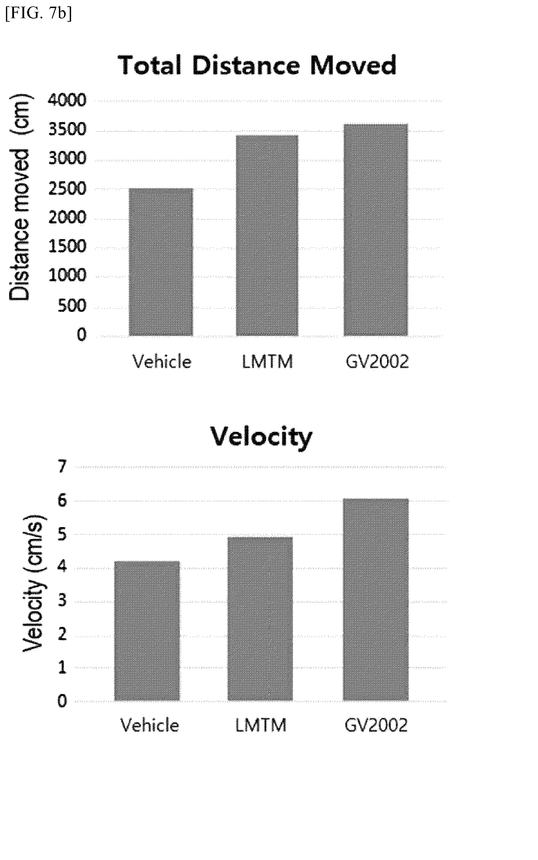

As shown in FIG. 7, the total distance traveled and movement speed in the open space were significantly higher in the GV2002-administered group compared to the vehicle-administered group, indicating that the motor ability of the GV2002-administered group was improved.

### 4-3. Evaluation of the Alleviation of Impairment in Cognitive Ability through the Novel Object Recognition Experiment

Novel object recognition experiments were conducted on a 4R tauopathy mouse model administered GV2002 for five months, from 7.5 to 12.5 months of age, to evaluate the efficacy of GV2002 in alleviating impairment in cognitive ability.

### (1) Experimental Method

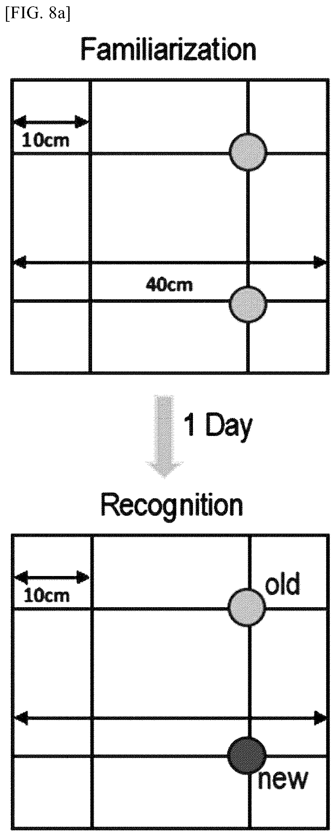

Mice that underwent the experiment from days 1 to 6 according to the experimental method in Example 4-2 were subjected to a novel object recognition experiment on days 7 and 8. On day 7, as shown in FIG. 8A, mice were placed in a 40 cm × 40 cm enclosure in which two similar objects were placed, and were allowed to explore for 10 minutes. On day 8, one of the two objects was replaced with a novel object, and mice were placed in the enclosure and observed for 10 minutes, during which the time spent exploring the novel object was measured. Prior to the experiments on days 7 and 8, mice were allowed one hour to acclimate to the testing environment. The illumination level was maintained at a constant level throughout the experiment. The recorded data were analyzed using two-way ANOVA with Sidak's multiple comparisons test.

### (2) Experimental Results

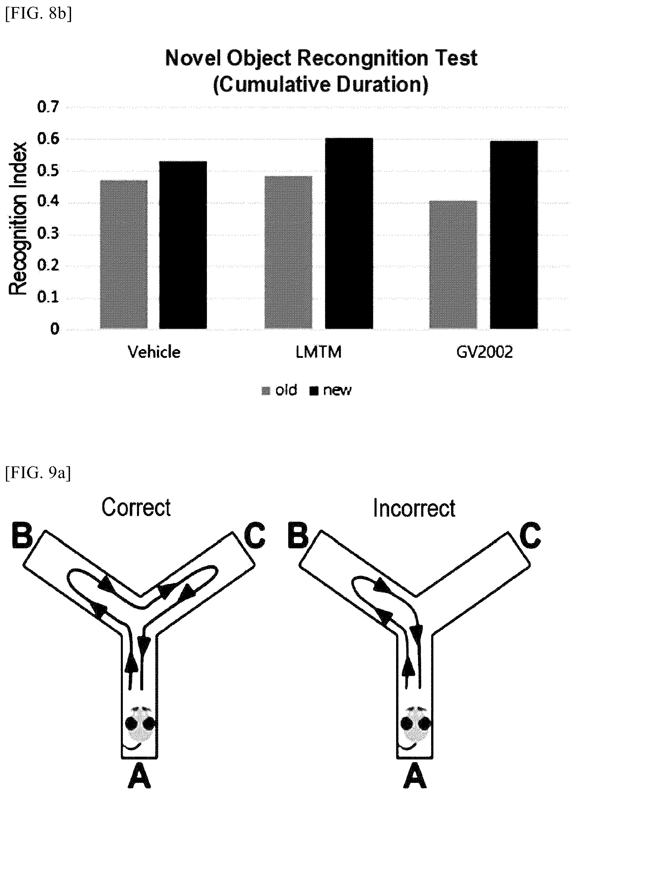

As shown in FIG. 8B, the vehicle-administered group failed to significantly distinguish between the learned object and the novel object, whereas the GV2002-administered group showed a significant increase in the time spent exploring the novel object compared to the time spent exploring the learned object, indicating that the cognitive ability of the GV2002-administered group was improved toward the novel object.

### 4-4. Evaluation of the Alleviation of Impairment in Spatial Memory through the Y-maze Experiment

Y-maze experiments were conducted on a 4R tauopathy mouse model administered GV2002 for five months, from 7.5 to 12.5 months of age, to evaluate the efficacy of GV2002 in alleviating impairment in spatial memory.

### (1) Experimental Method

Prior to the experiment, mice were allowed one hour to acclimate to the testing environment. Then, the mice were placed at starting point A of the Y-shaped platform, as shown in FIG. 9A. The mice's behavior was recorded for 8 minutes.

### (2) Experimental Results

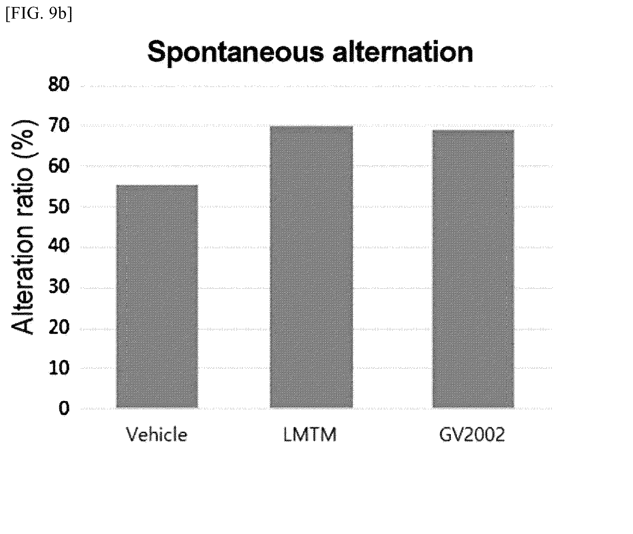

As shown in FIG. 9B, the GV2002-administered group showed a significant increase in the rate of spontaneous alternation behavior, which involved exploring a new area rather than a previously explored area, compared to the vehicle-administered group, indicating that the spatial memory of the GV2002-administered group was improved.

### 4-5. Evaluation of Tau Hyperphosphorylation Inhibition through Immunofluorescence Staining of Brain Tissue

The brains of a 4R tauopathy mouse model administered GV2002 for 5 months, from 7.5 to 12.5 months of age, were removed and analyzed using immunofluorescence staining.

### (1) Experimental Method

For brain tissue sampling, the 4R tauopathy mouse models were anesthetized, and saline was perfused intracardially, after which the blood was removed using a perfusion pump device. Then, the brains were removed and weighed. The brains to be used for tissue staining experiments were fixed in a 4% PFA solution for 24 hours. The fixed brain tissue was immersed in a 20% sucrose solution for 24 hours and then in a 30% sucrose solution for 48 hours for dehydration. The dehydrated brain tissue was removed, excess moisture was eliminated, and the brain tissue and a cryoembedding agent (OCT compound) were placed together in a mold to protect the tissue, after which the brain tissue was rapidly frozen using dry ice. The frozen brain tissue mold was stored in a -80 °C freezer. Afterwards, the brain tissue was sectioned into 30-µm-thick sections using a cryostat and stored in a 0.05% sodium azide solution.

For immunofluorescence staining of brain tissue, 30-µm-thick frozen brain sections were selected according to brain region, washed three times with PBS for 10 minutes each, fixed in 3.7% formaldehyde for 5 minutes, and washed three times with PBS for 10 minutes each. The brain tissue samples were permeabilized in 0.3% PBS-T solution, and then blocked in 5% BSA solution in PBS for 1 hour. The brain tissues were incubated overnight in 3% BSA and 0.1% Tween-20 solution in PBS containing AT8 (phospho-Tau S202/T205) antibody diluted at a ratio of 1:200, which targets tau hyperphosphorylation. The brain tissues were washed three times with PBS for 10 minutes each. The brain tissues were then incubated for 1 hour at room temperature in 3% BSA and 0.1% Tween-20 solution in PBS containing secondary antibody diluted 1:500. The brain tissues were immersed in PBS solution and washed once for 10 minutes. Nuclei were stained by immersing the brain tissues in Hoechst solution (stock concentration 1 mg/mL) diluted at a ratio of 1:2000 in PBS. The brain tissues were immersed in PBS solution and washed three times for 10 minutes each, and fluorescence imaging was performed using a slide scanner.

To obtain fluorescence images of the stained brain tissue, brain tissue slides were scanned using a Zeiss Axio Scan (Zeiss, Oberkochen, Germany). AT8 fluorescence levels in each brain tissue region obtained through fluorescence imaging, i.e., the somatosensory cortex, hippocampus (CA1), motor cortex, and substantia nigra, were calculated using ImageJ software (NIH). The calculated values were analyzed using one-way ANOVA with Dunnett's multiple comparisons test.

### (2) Experimental Results

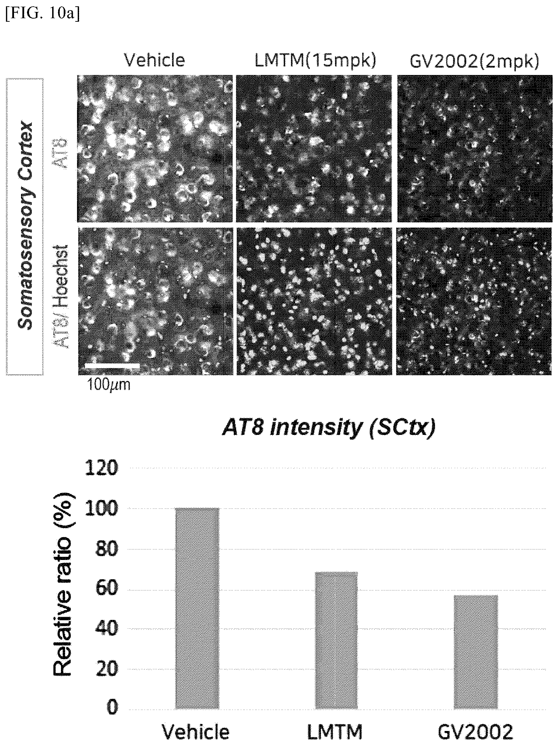

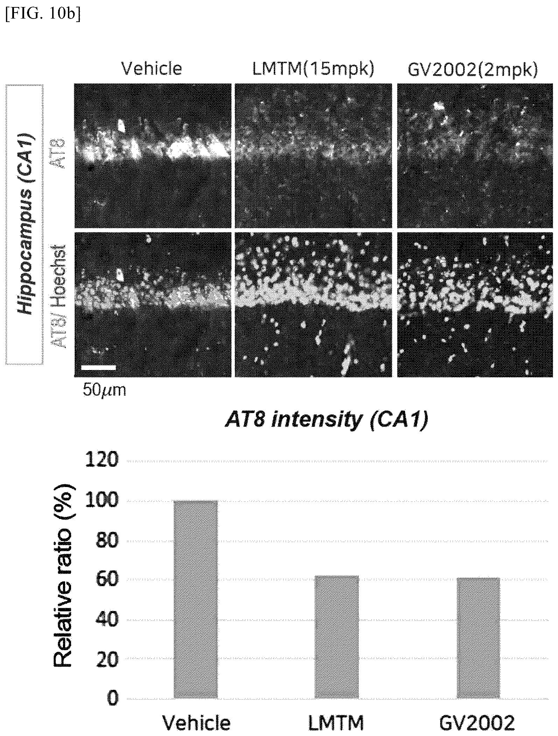

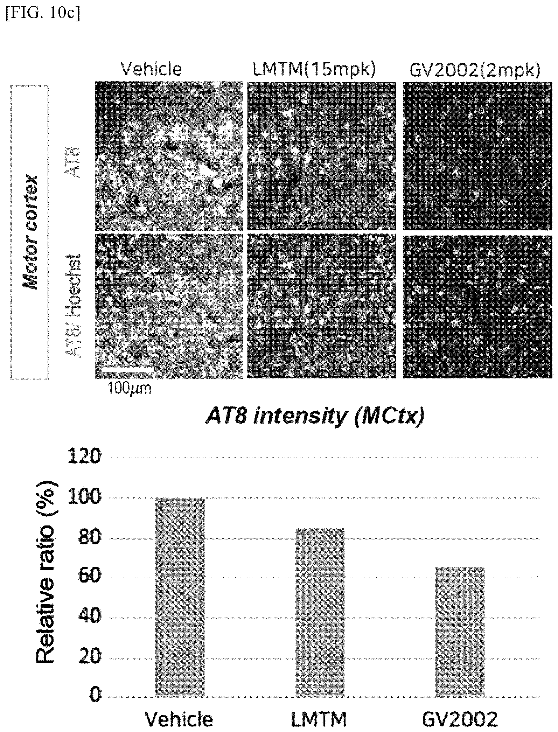

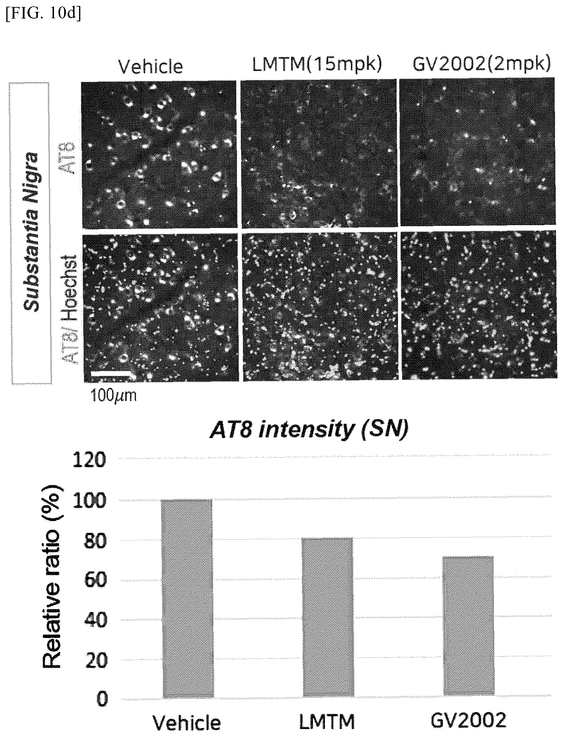

As shown in FIG. 10, tau hyperphosphorylation was significantly suppressed in all brain regions in the GV2002-administered group compared to the vehicle-administered group.

As shown in FIG. 10A, in the somatosensory cortex region, AT8 fluorescence intensity in the GV2002-administered group was significantly reduced by 57% compared to the vehicle-administered group.

As shown in FIG. 10B, in the hippocampus region, AT8 fluorescence intensity in the GV2002-administered group was significantly reduced by 62% compared to the vehicle-administered group.

As shown in FIG. 10C, in the motor cortex region, AT8 fluorescence intensity in the GV2002-administered group was significantly reduced by 65% compared to the vehicle-administered group.

As shown in FIG. 10D, in the substantia nigra region, AT8 fluorescence intensity in the GV2002-administered group was significantly reduced by 69% compared to the vehicle-administered group.

These results confirmed the inhibitory efficacy of GV2002 on 4R tau hyperphosphorylation.

### 4-6. Evaluation of Tau Oligomer Formation Inhibition through Brain Tau-BiFC Imaging

Brains from a 4R tauopathy mouse model administered GV2002 for five months, from 7.5 to 12.5 months of age, were removed and analyzed using lipid staining.

### (1) Experimental Method

Brain tissue sampling was performed in the same manner as in Example 4-5.

To remove autofluorescence from brain tissues through lipid staining, a 0.05% Sudan Black B solution in 70% ethanol was stirred overnight and then filtered through a 0.22-µm filter. 30-µm-thick frozen brain sections from each region were immersed in deionized water and washed three times for 5 minutes each. The brain tissue was then immersed in the prepared 0.05% Sudan Black B solution for 10 minutes to perform staining. The brain tissue stained with Sudan Black B was immersed in 0.1% PBS-T solution and washed at 30-second intervals to adjust the staining intensity and then washed three times with deionized water for 5 minutes each. Nuclear staining was performed by immersing the brain tissue in a 0.2-µg/mL Hoechst solution in deionized water for 20 minutes. The tissue was then washed three times with deionized water for 5 minutes each. Tau-BiFC imaging was performed using a slide scanner.

To obtain fluorescence images of the stained brain tissue, brain tissue slides were scanned using a Zeiss Axio Scan. Tau-BiFC fluorescence levels in each brain region obtained through fluorescence imaging, i.e., the somatosensory cortex, hippocampus (CA1), motor cortex, and substantia nigra, were calculated using ImageJ software. The calculated values were analyzed using one-way ANOVA with Dunnett's multiple comparisons test.

### (2) Experimental Results

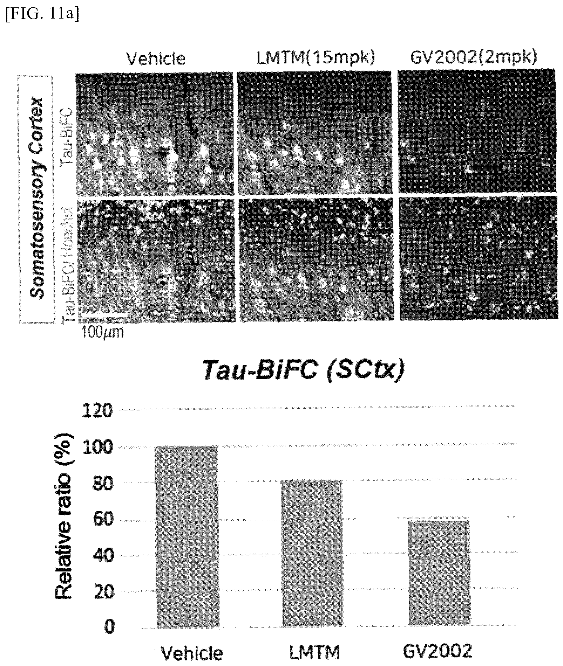

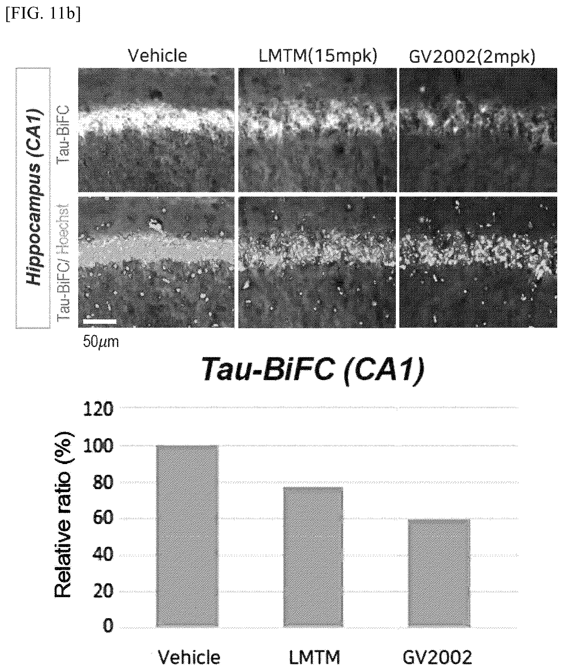

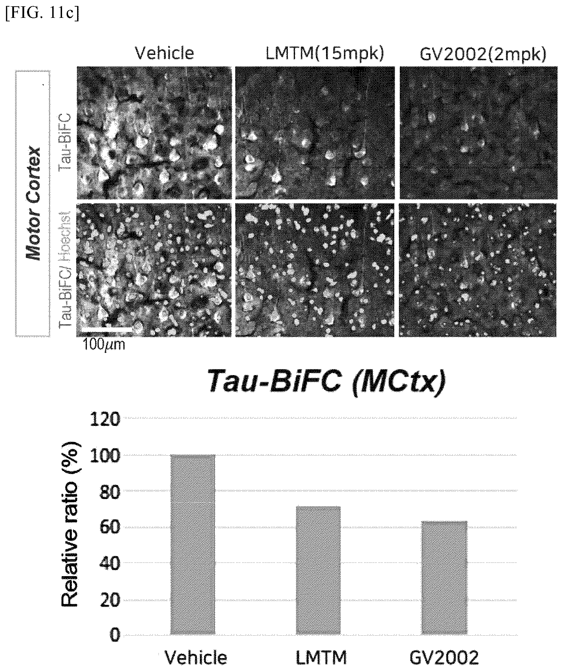

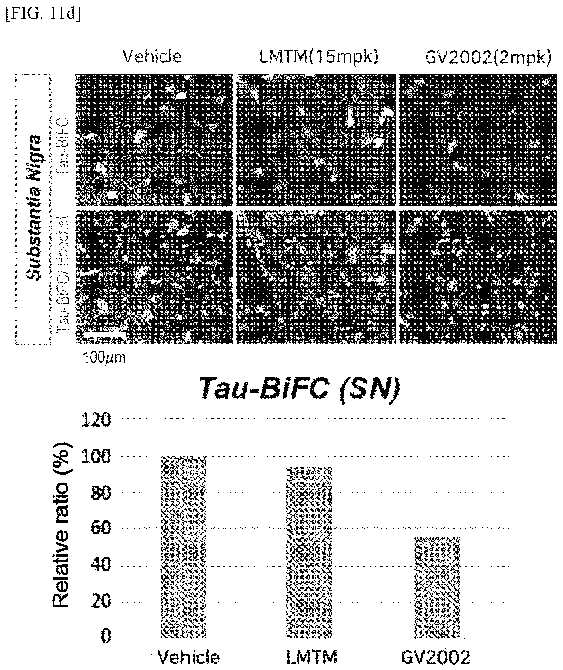

As shown in FIG. 11, tau oligomer formation was significantly inhibited in all brain regions in the GV2002-administered group compared to the vehicle-administered group.

As shown in FIG. 11A, in the somatosensory cortex region, the Tau-BiFC fluorescence intensity in the GV2002-administered group was significantly reduced by 58% compared to the vehicle-administered group.

As shown in FIG. 11B, in the hippocampus region, the Tau-BiFC fluorescence intensity in the GV2002-administered group was significantly reduced by 63% compared to the vehicle-administered group.

As shown in FIG. 11C, in the motor cortex region, the Tau-BiFC fluorescence intensity in the GV2002-administered group was significantly reduced by 56% compared to the vehicle-administered group.

As shown in FIG. 11D, in the substantia nigra region, the Tau-BiFC fluorescence intensity in the GV2002-administered group was significantly reduced by 56% compared to the vehicle-administered group.

These results confirmed the inhibitory efficacy of GV2002 on tau oligomer formation.

### 4-7. Evaluation of Tau Hyperphosphorylation and Aggregation Inhibition through Brain Lysate Analysis

Brain lysates from a 4R tauopathy mouse model administered GV2002 for five months, from 7.5 to 12.5 months of age, were obtained. Soluble and insoluble fraction samples were subjected to immunoblotting using antibodies against total tau (Tau5) and phosphorylated tau (pS199, pS396), and the levels of total and phosphorylated tau were quantitatively analyzed.

### (1) Experimental Method

Mouse models were anesthetized, saline was intracardially perfused, and blood was removed using a perfusion pump device. The brain was then removed and weighed. 1 mL of RIPA buffer (containing protease and phosphatase inhibitor cocktails) was added to the extracted brain and homogenized.

The homogenized brain lysates were transferred to EP tubes and incubated on an orbital shaker at 4°C for 2 hours. After centrifugation at 13,000 rpm for 20 minutes at 4°C, the supernatant (soluble fraction) was collected in a new EP tube and stored at -80°C.

To prepare samples for immunoblotting, the concentrations of the fractionated samples were quantified using the Bradford protein quantification assay. RIPA buffer and 4X SDS Laemmli sample buffer (containing 2.5% β-mercaptoethanol for reducing conditions) were added to each sample to adjust the final protein concentration to 2 mg/mL. The samples were then boiled at 97°C for 5 minutes.

Meanwhile, to obtain the insoluble fraction, the remaining pellet after supernatant removal was resuspended in RIPA buffer containing 1 M sucrose (1 mL) and DNase I (1 mg/mL). After centrifugation at 13,000 rpm for 20 minutes at 4°C, the supernatant was discarded, and the pellet was resuspended in 2% SDS solution (1 mL per 1 g of tissue) and incubated at room temperature for 1 hour. The mixture was centrifuged at 13,000 rpm for 1 minute at room temperature, and the resulting supernatant (insoluble fraction) was collected and transferred to a new EP tube. An equal volume of 2X SDS Laemmli sample buffer (containing 2.5% β-mercaptoethanol) was added, and the sample was boiled at 97°C for 5 minutes.

20 µg of each brain lysate sample of soluble and insoluble fractions were loaded onto a 10% SDS-PAGE gel (SDS-PAGE running buffer: 1X Tris/Glycine/SDS buffer in 3' D.W.), and electrophoresis was performed at 80 V for 30 minutes followed by 90 V for 1 hour. PVDF membranes trimmed to fit the transfer tray were soaked in 100% methanol for 1 minute, rinsed in autoclaved 3' D.W. for 3 minutes, and then incubated in chilled 1X transfer buffer (1X Tris/Glycine buffer with 20% methanol in 3' D.W.) for 10 minutes. Wet transfer was performed at 100 V for 1 hour and 20 minutes. The membranes were blocked in 5% BSA solution in TBS-T (containing 0.1% Tween-20) for 1 hour at room temperature. The membranes were then incubated overnight at 4°C on an orbital shaker in 2.5% BSA solution in TBS-T (containing 0.1% Tween-20) including primary antibodies: Anti-Tau (Tau5) antibody (ab80579, 1:5000), Anti-Tau (phospho S199) antibody (ab109390, 1:5000), and Anti-Tau (phospho S396) antibody (ab81268, 1:5000). The membrane was immersed in TBS-T (containing 0.1% Tween-20) and washed three times for 10 minutes each. The membranes were incubated for 1 hour at room temperature in 2.5% BSA solution in TBS-T including secondary antibodies: Goat Anti-Mouse IgG H&L (HRP) secondary antibody (ab6789, 1:10000) and Goat Anti-Rabbit IgG H&L (HRP) secondary antibody (ab6721, 1:10000). The membrane was again immersed in TBS-T (containing 0.1% Tween-20) and washed three times for 10 minutes each. Protein bands of the membrane were detected using ChemiDoc with ECL solution, which is HRP substrate, and band intensities were quantified using ImageJ software (NIH).

### (2) Experimental Results

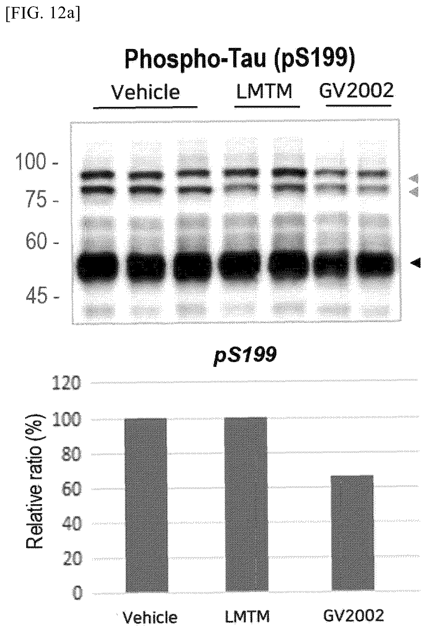

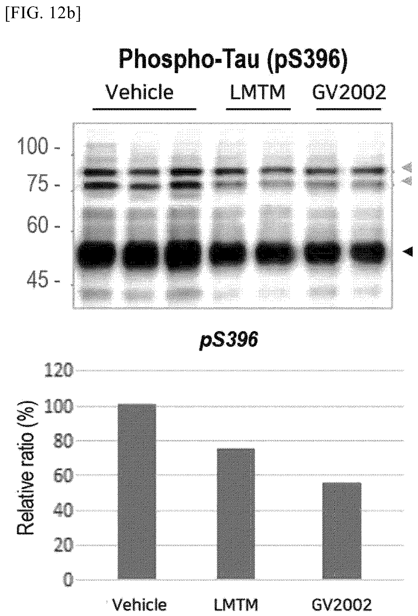

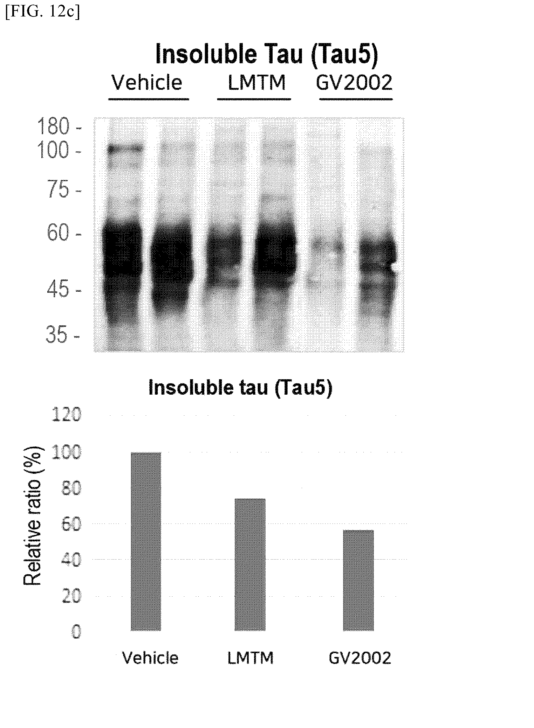

As shown in FIG. 12, the results of tau immunoblotting using the soluble fraction showed that the levels of phosphorylated tau pS199 and pS396 were significantly reduced in the GV2002-administered group compared to the vehicle-administered group. Tau immunoblotting using the insoluble fraction also showed that total tau levels were significantly reduced in the GV2002-administered group compared to the vehicle-administered group.

As shown in FIG. 12A, phosphorylated tau pS 199 in the GV2002-administered group was significantly reduced to 66% compared to the vehicle-administered group.

As shown in FIG. 12B, phosphorylated tau pS396 in the GV2002-administered group was significantly reduced to 56% compared to the vehicle-administered group.

As shown in FIG. 12C, the level of insoluble tau in the GV2002-administered group was significantly reduced to 57% compared to the vehicle-administered group.

These results confirmed that GV2002 exhibits tau phosphorylated form formation inhibitory efficacy and insoluble tau aggregation inhibitory efficacy.

## Claims

1. A peptide of Formula 1 below or a pharmaceutically acceptable salt thereof:

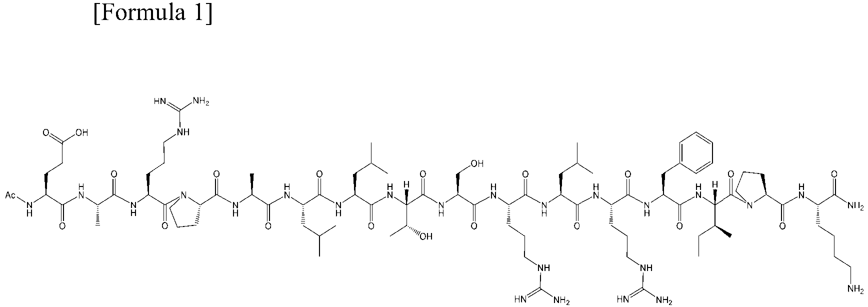

2. A pharmaceutical composition for preventing or treating 4R tauopathy, comprising the peptide of claim 1 or a pharmaceutically acceptable salt thereof.

3. The pharmaceutical composition according to claim 2, wherein the 4R tauopathy is any one selected from the group consisting of progressive supranuclear palsy (PSP), corticobasal degeneration (CBD), argyrophilic grain disease (AGD), globular glial tauopathy (GGT), and aging-related tau astrogliopathy (ARTAG).

4. The pharmaceutical composition according to claim 3, wherein the prevention or treatment of 4R tauopathy is achieved by inhibiting hyperphosphorylation and aggregation of tau protein.

5. A health functional food for preventing or alleviating 4R tauopathy, comprising the peptide of Formula 1 below or a food-acceptable salt thereof:

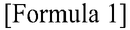

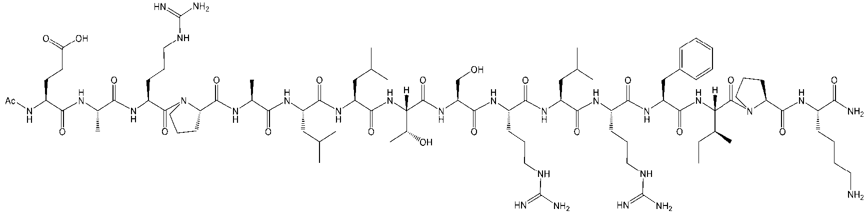

6. The health functional food according to claim 5, wherein the 4R tauopathy is any one selected from the group consisting of progressive supranuclear palsy, corticobasal degeneration, argyrophilic grain disease, globular glial tauopathy, and aging-related tau astrogliopathy.

7. The health functional food according to claim 6, wherein the prevention or alleviation of 4R tauopathy is achieved by inhibiting hyperphosphorylation and aggregation of tau protein.
